(19) Europäisches Patentamt / European Patent Office / Office européen des brevets

(11) **EP 3 675 129 A1**

(12) **EUROPEAN PATENT APPLICATION**

(43) Date of publication:
**01.07.2020 Bulletin 2020/27**

(51) Int Cl.:
***G16B 30/00*** (2019.01)

(21) Application number: **18248105.1**

(22) Date of filing: **27.12.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **IMEC vzw**
  **3001 Leuven (BE)**
• **Universiteit Gent**
  **9000 Gent (BE)**

(72) Inventors:
• **FOSTIER, Jan**
  **3001 LEUVEN (BE)**
• **STEYAERT, Aranka**
  **3001 LEUVEN (BE)**
• **AUDENAERT, Pieter**
  **3001 LEUVEN (BE)**

(74) Representative: **IP HILLS NV**
  **Hubert Frère-Orbanlaan 329**
  **9000 Gent (BE)**

(54) **PROCESSING READS OF SEQUENCED NUCLEIC ACID**

(57) Example embodiments relate to a method for processing reads (502) of sequenced nucleic acid (500) comprising: i) determining (503) k-mers (504, 551); ii) determining (505) first counts (553) indicative for the respective number of occurrences of the respective k-mers (551), and second counts (554) indicative for the respective number of overlaps (560) between respective k-mers; iii) determining (507) a k-mer spectrum (530) from the first counts; iv) determining (507) first output counts indicative for the respective number of occurrences of the respective k-mers in the nucleic acid based on a position (531, 532, 533) of the k-mers in the k-mer spectrum (530) and based on a constraint (509, 510). The constraint comprising: i) a first output count of a respective k-mer equals the total number of overlaps of the respective k-mer with other k-mers in the nucleic acid; and/or ii) a first output count of a respective k-mer equals the total number of overlaps of other k-mers with the respective k-mer.

Fig. 5

**Description**

**Technical Field**

Various example embodiments relate to processing of reads of a sequenced nucleic acid such as a DNA or RNA sequence. More particular, they relate to determining the number of occurrences of respective k-mers in the nucleic acid from the reads.

**Background**

When sequencing a DNA sequence, the order of nucleotides in the DNA sequence is determined. As there are four different nucleotides: adenine (A), cytosine (C), guanine (G) and thymine (T), the result of the sequencing may be represented as a string composed with letters of the four letter alphabet A, C, G and T.

Illumina dye sequencing is a popular sequencing technique because it offers a high throughput and low sequencing cost. The output of the Illumina dye sequencing are short reads wherein a single read represents a short subsequence, e.g. 100 to 250 nucleotides, of the DNA sequence. The output does not contain positional information for the reads, i.e., the location of the reads within the DNA sequence is unknown. Each nucleotide within the DNA sequence is typically covered by multiple fully or partially overlapping reads. The average number of reads that cover a nucleotide in the DNA sequence, the sequencing coverage, typically ranges from 30 to 50.

A problem with Illumina dye sequencing is that the reads contain sequencing errors, e.g. substitutions, insertions and deletions. The error rate is typically in the range of 1% to 2%, the vast majority being substitutions.

Different techniques have been developed to derive information on the DNA sequence from such error-containing reads. One such technique uses a directed graph representation called a de Bruijn graph. According to this technique, k-mers are first derived from the reads, i.e., all possible sequences with a length of k nucleotides appearing in the reads. Each unique k-mer is then represented as a node of the graph wherein each node is associated with the number of occurrences of the respective k-mer in the reads, the read support of the node. Similarly, the number of k-1 overlaps from one k-mer to another one, the read support of an arc, is associated with the arc between the respective k-mers. The read support of the nodes and the arcs is then used to infer the occurrences of the k-mers in the original DNA sequence, further referred to as the multiplicities of the nodes and arcs. This inference may be based on the so-called k-mer spectrum, i.e. a histogram of the number of k-mers as a function of their read support. From this spectrum, thresholds are derived for classifying the read support of the nodes into multiplicities.

A problem with the above technique is that the k-mer spectrum is a superposition of distributions corresponding to the different multiplicities. Because these distributions overlap, the multiplicities may be erroneously assigned. For example, an erroneous k-mer may be assigned a multiplicity of one because it occurs more often than average in the reads.

**Summary**

It is an object, amongst others, to alleviate the above identified shortcomings and to provide a solution for processing reads of a sequenced nucleic acid that results in fewer errors in the obtained multiplicities.

According to a first aspect, this object is achieved by a computer-implemented method for processing reads of a sequenced nucleic acid comprising the following steps: i) determining k-mers from the reads; ii) determining first counts indicative for the respective number of occurrences of the respective k-mers in the reads, and second counts indicative for the respective number of overlaps between respective k-mers in the reads; iii) determining a k-mer spectrum from the first counts; iv) determining first output counts indicative for the respective number of occurrences of the respective k-mers in the nucleic acid based on a position of the k-mers in the k-mer spectrum and based on a constraint. The constraint comprising: i) a first output count of a respective k-mer equals the total number of overlaps of the respective k-mer with other k-mers in the nucleic acid; and/or ii) a first output count of a respective k-mer equals the total number of overlaps of other k-mers with the respective k-mer.

In other words, when representing the k-mers by a directed graph, the first counts would correspond to the read support of the nodes and the second counts would correspond to the read support of the directed edges, the arcs, between the respective nodes. The first output counts then correspond with the determined multiplicity of the k-mers in the nucleic acid. According to the above method, the output counts are not solely based on the k-mer spectrum, i.e. on a fixed threshold obtained from the k-mer spectrum. A further constraint is applied taking into account the neighbourhood of the respective k-mer. When representing the k-mers by a directed graph, this constraint imposes that the directed graph is a balanced directed graph, i.e., that for each node, its multiplicity is equal to the sum of the multiplicities of its incoming arcs and equal to the sum of the multiplicities of its outgoing arcs. This improves the statistical power of assigning multiplicities to nodes or arcs. Specifically, it also improves the accurate identification of nodes or arcs with multiplicity zero, and thus in a better identification of sequencing errors in the reads.

The above method may further comprise determining second output counts indicative for the respective number of overlaps between respective k-mers in the nucleic acid based on the position of the k-mers in the k-mer spectrum and based on the constraint. In other words, also the multiplicities of the arcs may be derived in a similar way.

According to an example embodiment, the first and/or second output counts may be determined by assigning probabilities for candidate output counts. In other words, as the k-mer spectrum already provides information on the probabilities for a certain multiplicity of a node or arc based on the read support, the constraint may be used to further adapt the probabilities on a per node or arc basis. A candidate output count is then assigned a lower probability when violating the constraint.

According to an embodiment, the determining the first and/or second output counts is performed by a probabilistic graphical model. Such a model allows deriving the constraint based multiplicities. The model may for example be a conditional random field, CRF. This way, the output counts may be obtained by iteratively estimating the parameters of the k-mer spectrum and the node- and arc multiplicities in an expectation-maximization setting. More generally, the first output counts and/or second output counts may be the unobserved variables of the model. Similarly, the first and/or second counts are the observed variables of the model.

According to a further embodiment, the determining the first and/or second output counts further comprises selecting a neighbourhood of a respective k-mer and constructing a probabilistic graphical model of the neighbourhood and determining the first output count and/or the second output count associated with the respective k-mer therefrom.

By limiting the number of nodes to a neighbourhood of the k-mer, the number of random variables in the model is reduced. Moreover, as the multiplicities of the neighbouring node have the biggest impact on the constraint, the output counts are obtained faster without a significant loss in accuracy.

According to various example embodiments, the overlaps between the k-mers are k-1 overlaps.

According to a second aspect, the invention relates to a data processing system configured to perform the method according to the first aspect.

According to a third aspect, the invention relates to a computer program comprising instructions which, when the program is executed by a computer, cause the computer to perform the method according to the first aspect.

According to a fourth aspect, the invention relates to a computer-readable medium comprising instructions which, when executed by a computer, cause the computer to perform the method according to the first aspect.

## Brief Description of the Drawings

Some example embodiments will now be described with reference to the accompanying drawings.

Fig. 1 illustrates a DNA sequence, reads obtained therefrom, read coverage depth, and errors in the reads; and

Fig. 2 illustrates an example DNA sequence and a corresponding directed graph representation of the DNA sequence; and

Fig. 3 illustrates example reads obtained from the example DNA sequence and a corresponding directed graph representation of the reads; and

Fig. 4 illustrates example reads containing read errors obtained from the example DNA sequence and a corresponding directed graph representation of the reads containing the errors; and

Fig. 5 illustrates steps performed according to an example embodiment to obtain node and arc multiplicities of a DNA sequence from reads; and

Fig. 6 illustrates a directed graph containing node and arc multiplicities of a DNA sequence obtained by the steps illustrated in Fig. 5; and

Fig. 7 illustrates a directed graph representation of a DNA sequence obtained by a method according to the state of the art; and

Fig. 8 illustrates a probabilistic graphical model for obtaining node and arc multiplicities of a DNA sequence from reads according to an embodiment of the invention; and

Fig. 9 shows an example embodiment of a suitable computing system for performing steps according to example aspects of the invention.

## Detailed Description of Embodiment(s)

Various example embodiments relate to processing of reads of a sequenced nucleic acid. More particular, they relate to determining the number of occurrences of respective k-mers in the nucleic acid from the reads. The below description uses a deoxyribonucleic acid, or shortly DNA sequence, as an example of a nucleic acid but the below description may also be applied to other forms of nucleic acid such as a ribonucleic acid, or shortly RNA sequence.

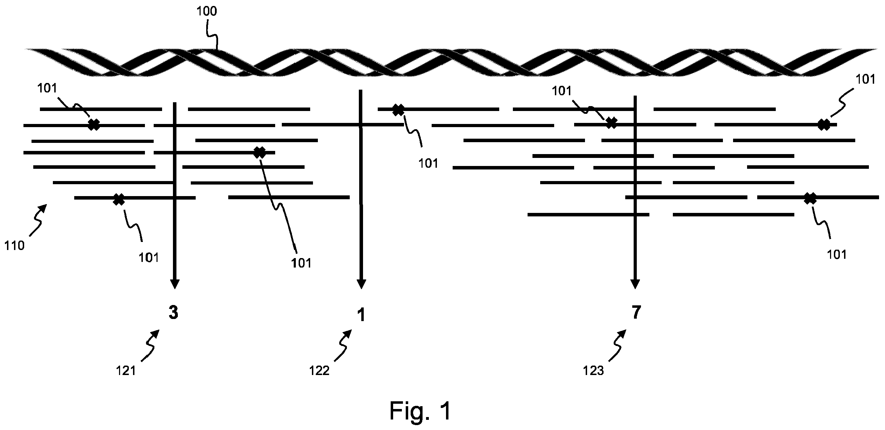

Fig. 1 illustrates a DNA sequence 100 and reads 110 obtainable by sequencing the DNA sequence 100, e.g. by Illumina dye sequencing. Such a read 110 represents a short subsequence, e.g. 100 to 250 nucleotides, of the DNA sequence 100. The output of the sequencing does not contain positional information for the reads 110, i.e., the location of the reads 110 within the original DNA sequence 100 is unknown. Each nucleotide within the DNA sequence 100 is typically covered by multiple fully or partially overlapping reads 110. The average number of reads that cover a nucleotide in the DNA sequence, the coverage 121, 122, 123, typically ranges from 30 to 50. Due to the stochastic nature of the sequencing, the coverage of a nucleotide may vary. Fig.1 illustrates this by way of an example wherein different nucleotides have a coverage of respectively three, one and seven. The reads 110, may further comprise sequencing errors 101 such as substitutions wherein a read comprises a different nucleotide in comparison with the DNA sequence 101; a deletion wherein a nucleotide is missing in a read in comparison with the DNA sequence 100; and an insertion wherein an extra nucleotide is present in the read in comparison with the DNA sequence. The error rate for Illumina dye sequencing is typically in the range of 1% to 2% wherein the vast majority of the errors are substitutions.

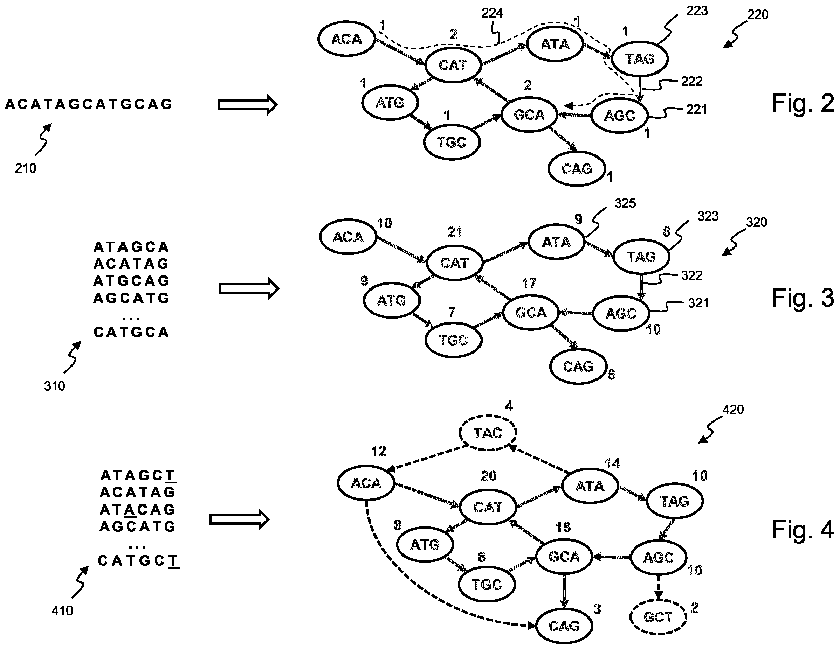

Fig. 2 illustrates a representation of DNA sequence 210 in the form of a de Bruijn graph 220, i.e. a directed graph with nodes 221 and arcs 222 each being assigned a number or count. A node 221 of the de Bruijn graph represents a k-mer of a DNA sequence 210, i.e. a subsequence with a length of k nucleotides. An arc 222 of the de Bruijn graph between two nodes 223, 221 represents an overlap with a predefined number of nucleotides between the two nodes 223, 221. Typically for a de Bruijn graph, the overlap is (k - 1) nucleotides. As the original DNA sequence 210 has a direction, i.e. a start and an end nucleotide, the arcs also have a direction, representing the reading direction of the string representation of the DNA sequence 210. A node 223 is further associated with a node count which is indicative for the respective number of occurrences of the respective k-mer in the DNA sequence 210. For example, the k-mer 'TAG' of node 223 occurs one time in the DNA sequence 210. Similarly, an arc 222 is further associated with an arc count (not shown in the figure) which is indicative for the respective number of occurrences of overlaps between the connecting nodes, i.e. between the connecting k-mers. For example, the overlap 'TAGC' 222 between the k-mer 'TAG' 223 and the k-mer 'AGC' 221 occurs one time in the DNA sequence 210. When a de Bruijn graph 220 is built from a DNA sequence 210, the underlying sequence is present in the graph 220 as a path 224 through the graph. A node or arc count in a de Bruijn graph that represents a DNA sequence 210 is also referred to as the node or arc multiplicity.

Similarly, a de Bruijn graph may be constructed from the reads 110 obtained from a DNA sequence 100. This is illustrated in Fig. 3, wherein example reads 310 obtained from DNA sequence 210 are shown. In the example of Fig. 3, no sequencing errors are present in the reads 310. On the right side, Fig. 3 illustrates a de Bruijn graph 320 constructed from the reads 310. As the reads 310 are directly obtained from DNA sequence 210, the node and arc counts of graph 320 are related to the node and arc multiplicities of graph 220. As the read coverage is not constant, this relationship will not be strictly linear. For example, nodes 322, 323 and 325 have a different node count while they all relate to a node multiplicity of one.

Furthermore, by the introduction of read errors, different erroneous nodes and arcs may be introduced in the de Bruijn graph. This is illustrated in Fig. 4 showing a de Bruijn graph 420 constructed from reads 410 obtained from sequencing DNA sequence 210 wherein the reads 410 contain various errors (underlined in Fig. 4). Because of these errors, additional arcs and nodes are introduced as illustrated by the dashed lines in graph 420.

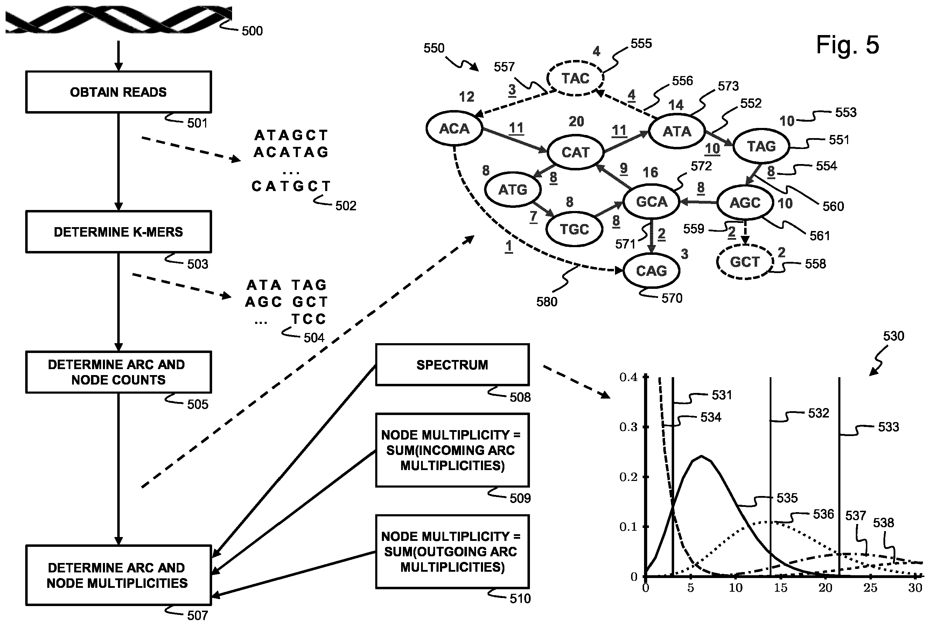

The below example embodiments describe how to process the reads 410 of a sequenced DNA sequence 210 in order to assign the node and/or arc multiplicities. To this respect, Fig. 5 illustrates steps of a method for processing such reads according to an embodiment of the invention. In a first step 501, reads 502 of a DNA sequence 500 are obtained. The reads 502 may for example be obtained by Illumina dye sequencing or any other suitable method. Reads 502 are used as an example for describing the steps. Reads 502 corresponds to the example of Fig. 4 and contain various read errors. In a next step 503, k-mers 504 are determined from the reads 502. According to the illustrative example of Fig. 5, k equals three resulting in k-mers 504: 'ATA', 'TAG', 'AGC'... In real-life circumstances, k is preferably much larger, e.g. from 50% to 70% of the read length with absolute values ranging e.g. from 15 to 50.

At a next step 505, the arc and node counts 553, 554 of the k-mers are determined. In other words, the de Bruijn graph 550 is determined. Although the counts determine the de Bruijn graph, it is not essential that a graph 550 is constructed. The node and arc counts may for example also be stored in a table or database thereby representing the de Bruijn graph 550. Due to the errors in the reads, different errors are introduced in the graph 550. For example, a new intermediate node 555 and end node 558 is introduced together with connecting arcs 556, 557 and 559. Also, a new arc 580 is introduced.

Then, in a next step 507, the arc and/or node multiplicities are determined based on the k-mer spectrum 508, 530 and based on the following constraints 509, 510: i) the multiplicity of a node should equal the sum of the multiplicities of the outgoing arcs of that node; and ii) the multiplicity of a node should equal the sum of the multiplicities of the arcs entering the node. This constraint relates to an inherent property of the de Bruijn graph representing the original DNA sequence.

An example 530 of a k-mer spectrum is also shown in Fig. 5. The k-mer spectrum may be constructed by determining, for each possible k-mer node count, the number of k-mers 504 that have that respective node count. This results in a spectrum 530, i.e., the distribution of node counts. This is illustrated in plot 530 by a mixed distribution comprising sub-distributions 534-538 for each respective node multiplicity, i.e. distribution 530 is the sum of a first sub-distribution 534 of k-mers with node multiplicity zero, a second sub- distribution 535 for k-mers with node multiplicity one, a third sub-distribution 536 for k-mers with node multiplicity two, a fourth sub-distribution 537 for k-mers with node multiplicity three etc. These sub-distributions are not known but may be estimated from the obtained distribution by a fitting operation. With the estimated sub-distributions, for each node count, and thus for each node, a probability may be derived that the respective node is an error, has a multiplicity one, a multiplicity two etc.

One way to determine the multiplicities therefrom is to determine hard multiplicity intervals 531, 532, 533 etc. each indicating a count interval within which a k-mer is assigned a certain multiplicity. However, as will be demonstrated further below, this solution is not ideal. Therefore, according to step 507, the constraints 509, 510 are further taken into account in assigning the multiplicities. Advantageously, this is done by, for each node and arc, assigning probabilities for the different multiplicities based on the k-mer spectrum and, additionally, increasing or decreasing the probability based on the how good the graph fulfils the constraint. In other words, each node is assigned a set of candidate output counts, each with a certain probability based on the k-mer spectrum. These probabilities are then further adjusted according to the constraints 509, 510. From these probabilities, a decision on the multiplicity of each node and arc may be taken.

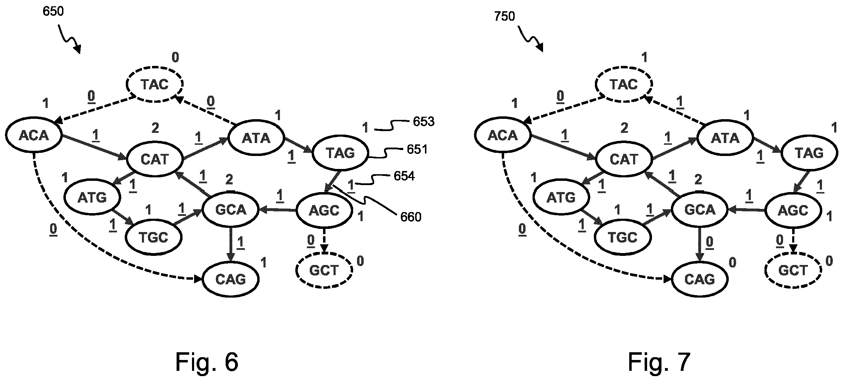

Fig. 6 illustrates a de Bruijn graph 650 with node and arc multiplicities determined from the graph 550 based on the steps of Fig. 5. Fig. 7 on the other hand, illustrates a de Bruijn graph 750 with node and arc multiplicities determined from the graph 550 when only considering the k-mer spectrum 530, i.e. by deciding the multiplicity according to fixed thresholds 531, 532, 533.

Regarding node 570, based on the k-mer spectrum cutoff 531, the node 570 and arc 571 would be assigned multiplicity zero as shown in Fig. 7. On the other hand, when further taking into account the constraints 509, 510, assigning multiplicity zero to node 570 would further violate the constraint on node 572. Therefore, the probability that node 570 has multiplicity one is higher than the probability that is has multiplicity zero. A similar consideration applies to node 555. According to the k-mer spectrum, node 555 would be assigned multiplicity one as shown in Fig. 7. However, as the node count is four, the probability that node 555 has multiplicity one is only slightly higher than the probability that it has multiplicity zero. Furthermore, assigning it with multiplicity one would further violate the constraint on node 573 which already has a high probability for multiplicity one for both the node and arcs. Hence, according to step 507, it is more likely that node 555 is due to an error and receives multiplicity zero.

Different techniques may be used to apply the constraints 509 and 510 in the determination of the multiplicities. According to an example embodiment, a probabilistic graphical model, more particular a conditional random field, CRF, model, is used. When determining multiplicities of nodes and arcs while also taking into account constraints 509, 510, information of neighbouring nodes and arcs must be taken into account as demonstrated in the simplified example above. This results in a high dimensional problem. By using conditional random fields, this high dimensional problem is transformed into probabilities that are products of factors and independencies are more easily represented. Moreover, efficient computational methods may be used to infer the individual probabilities present in the model. The CRF model allows combining the information contained in the k-mer spectrum 530 with constraints 509, 510.

A CRF model is a type of Probabilistic Graphical model or graph model wherein the nodes represent variables and the edges represent direct probabilistic interactions between these variables. These variables are further divided into a set of observed variables and a set of unobserved variables from which information is inferred. When using a CRF model for performing step 507, the observed variables $\mathbf{X} = \{X_1,..., X_m\}$ may correspond to the coverage of arcs in the de Bruijn graph 550 and the unobserved variables $\mathbf{Y} = \{Y_1,...,Y_m\}$ may correspond to the multiplicities of nodes and arcs in the resulting de Bruijn graph 650. Probabilistic interactions may then be represented by factors $\varphi_i(\mathbf{D}_i)(\mathbf{D}_i \subseteq \mathbf{X} \cup \mathbf{Y}; \mathbf{D}_i \not\subseteq \mathbf{X})$ such that all variables in the scope $\mathbf{D}_i$ of $\varphi_i$ form a clique in the CRF. The joint conditional probability over all variables may then be calculated as

$$P(\mathbf{Y}|\mathbf{X}) = \frac{1}{Z(\mathbf{X})} \prod_{i=1}^{M} \varphi_i(\mathbf{D}_i)$$

wherein $Z(\mathbf{X})$ is the partition function that normalises the product of factors such that $P(\mathbf{Y}|\mathbf{X})$ is a probability distribution. By performing the conditioning on $\mathbf{X}$, the modelling of dependencies between these variables may be avoided and only the conditional dependencies between the $\mathbf{Y}$s are modelled. Two types of factors may further be discerned in the model. The first type, referred to as the "singleton factors" $\varphi_a$, models the relationship between the multiplicity of the arcs in the de Bruijn graph and their observed k-mer coverage. The second type, referred to as "conservation of flow factors" $\varphi_{\text{flow}}$, impose the conservation of flow according to the constraints 509 and 510 through the resulting de Bruijn graph 650. Edges connect nodes such that all variables that are in the scope $\mathbf{D}_i$ of a factor, form a clique in the CRF. The two different factor types will pull the belief, i.e. probability for a certain multiplicity in the graph, in a certain direction. In other words, the final multiplicity-probabilities will be the result of an interplay between the observed k-mer coverage in graph 550, because of $\varphi_a$, and an implied conservation of flow according to constraints 509, 510, because of $\varphi_{\text{flow}}$. In some cases, both may point to the same multiplicity. In other cases, $\varphi_a$ will put more belief in multiplicity = 1, while $\varphi_{\text{flow}}$ imposes the multiplicity to be equal to zero, e.g. in case of a highly covered error, or the other way around, e.g. in case of a low coverage region.

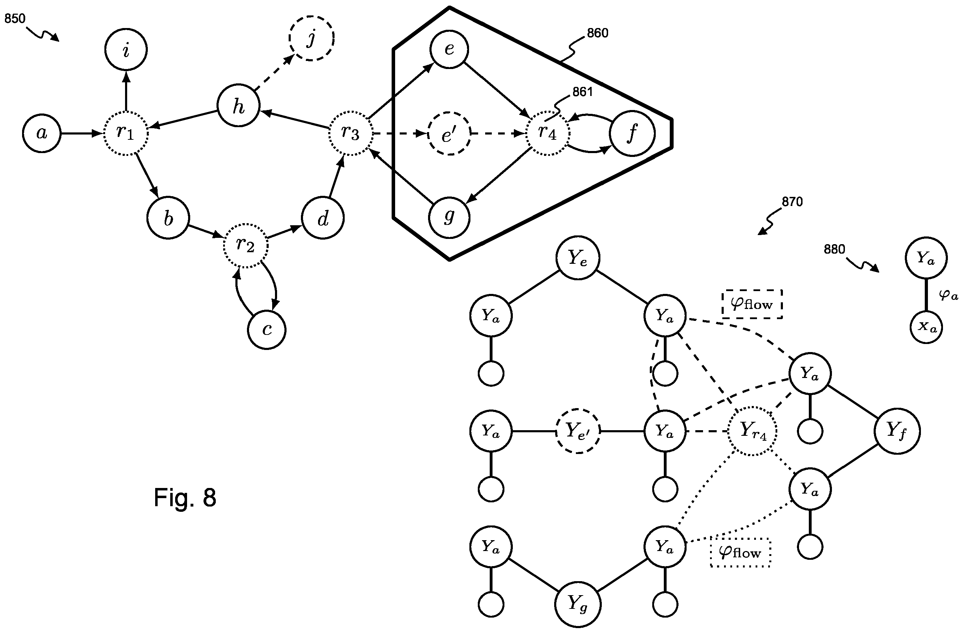

According to an embodiment, the CRFs are built for small regions of the de Bruijn graph. This way, computational complexity is greatly reduced compared with the case where the whole de Bruijn graph is taken into account. This is further illustrated in Fig. 8. To infer the multiplicity of a certain node 861 in a de Bruijn graph 850, a neighbourhood 860 around this node 861 is selected. Such a neighbourhood of size s comprises all nodes that are reachable by a path of length $\leq s$ from node 860 and all of its incoming and outgoing arcs. For each node m in the neighbourhood 860 the CRF contains a variable node $Y_m$. For each arc $a$ in the neighbourhood the CRF contains variable nodes $Y_a$ and $X_a$. The de Bruijn graph 850 with a selected neighbourhood 860 illustrates the case wherein $s = 1$ together with the associated CRF 870.

For each $X_a$ and $Y_a$ corresponding to an arc in the de Bruijn graph, a "singleton factor" 880

$$\varphi_a(Y_a, X_a) : Val(Y_a, X_a) \mapsto \mathbb{R}^+$$

is created that represents the probability that an arc has multiplicity $m$ given that a coverage $C$ is observed. Because the time and memory requirements of the subsequent inference algorithms depend on the number of possible values for the variables, $Val(Y_a)$ is restricted to $[max(0, m_{opt} - \alpha), m_{opt} + \alpha]$ wherein $\alpha$ is a tuneable parameter, e.g. $\alpha = 2$, and $m_{opt}$ is the current estimation for the most likely multiplicity of $Y_a$. To estimate these probabilities, a mixed model of negative binomials may be fit based on the k-mer spectrum 530 of the data:

$$\varphi_a(m, C) = \begin{cases} w_m P(X = C), & X \sim \mathcal{NB}(m\lambda, fm\lambda) & m > 0 \\ w_0 P(X = C), & X \sim \mathcal{NB}(\lambda_{\text{err}}, f\lambda_{\text{err}}) & m = 0 \end{cases}$$

For this mixed model some parameters are to be estimated: $\lambda_{\text{err}}$, the mean coverage of the erroneous k-mers, i.e. those with multiplicity 0, and $\lambda$, the mean coverage of k-mers with multiplicity 1 in the DNA sequence. For each multiplicity with $m > 1$ the negative binomial has mean $m\lambda$. Each negative binomial may further be weighted according to the estimated number of k-mers with that multiplicity in the dataset, $w_m$. To determine the variance of a negative binomial an overdispersion factor f may be used such that the variance of a negative binomial with mean $\lambda$ is equal to $f\lambda$.

For each $Y_n$ corresponding to a node $n$ in the de Bruijn graph, two "conservation of flow factors" are created. Each node generates a conservation of flow factor for the incoming arcs and one for the outgoing arcs. The scope of these factors then contains the corresponding CRF-node $Y_n$ and all $Y_a$ corresponding to incoming respectively outgoing arcs of node $n$, i.e., for the incoming arcs:

$$\varphi_{\text{flow}}(m_n, m_a, \ldots, m_a) = \begin{cases} 1, & \sum_{a \in \text{In}(n)} m_a = m_n \\ \varepsilon, & \sum_{a | \in \text{In}(n)} m_a \neq m_n \end{cases}, \varepsilon \ll 1$$

These factors

$$\varphi_{\text{flow}}(Y_n, Y_a, \ldots, Y_a) : Val(Y_n, Y_a, \ldots, Y_a) \mapsto \mathbb{R}^+$$

assign a value of 1 to combinations of values such that the sum of the multiplicities of the incoming respectively outgoing arcs is equal to the multiplicity of the node and a low value (e.g. $10^{-7}$) otherwise. While the singleton factors represent the knowledge present in each node and arc individually, the conservation of flow factors will enforce a consistent assignment (following a conservation of flow of multiplicity) over all nodes and edges represented in the CRF. An analogous equation may be constructed for the outgoing arcs.

The product of all factors is the joint probability of the multiplicities over all nodes and arcs in the selected neighbourhood 860. To determine the probability distribution over the possible multiplicities of a node $n$ (or similarly an arc $a$) the result of the following equation is needed:

$$P(Y_n|\mathbf{X}) = \sum_{\mathbf{Y}\backslash Y_n} P(\mathbf{Y}|\mathbf{X})$$

This may be done using a variable elimination algorithm performing an exact calculation of these probabilities.

The values of the factors in the above CRF depend on parameters which also need to be estimated. These estimates depend on the multiplicities of the nodes and arcs in the de Bruijn graph, which are also unknown. Therefore, the CRF-model may be used in an Expectation-Maximisation setting as follows:

    1) Use data heuristics to find a first estimate for these parameters.

    2) Estimate (E) the most likely multiplicities for the nodes and arcs of the de Bruijn graph, using a CRF with factors based on the current parameters.

    3) Estimate (M) new values for the parameters using the newly inferred multiplicities, weighted by their probability.

Convergence is then reached when the estimated multiplicity of a certain percentage of the nodes and arcs does not change between different E-steps.

The parameters in the CRF that need to be determined are: i) the means of the negative binomial distributions $\lambda$ and $\lambda_{err}$, ii) the overdispersion factor $f$ determining the variance of the negative binomial, and iii) the weights $w_i$ for possible multiplicities. To calculate the values of these parameters the following values derived from the data may be used: cov($n$) denoting the k-mer coverage of a node $n$; cov($a$) denoting the k-mer coverage of an arc $a$; wherein the representation of the de Bruijn graph concatenates nodes that have only one incoming and one outgoing arc, i.e. cov($n$) is the total k-mer coverage of all k-mers in the concatenated node. The set of all k-mers in node $n$ is further denoted by k-mers($n$), and its size by |k-mers($n$)|. The current multiplicity estimate for nodes and arcs is denoted by mult($n$) and mult($a$) respectively. Since nodes correspond to (concatenated) k-mers, while the arcs correspond to (k - 1)-mers, the underlying distributions will differ slightly. Therefore, all parameters are estimated separately for the nodes and the arcs of the de Bruijn graph.

The negative binomial means may be estimated as follows:

$$\hat{\lambda}^{\text{node}} = \frac{\sum\limits_{\substack{\text{nodes } n \\ \text{mult}(n)>0}} \text{cov}(n)}{\sum\limits_{\substack{\text{nodes } n \\ \text{mult}(n)>0}} |\text{k-mers}(n)|\text{mult}(n)} \qquad \hat{\lambda}^{\text{arc}} = \frac{\sum\limits_{\substack{\text{arcs } a \\ \text{mult}(a)>0}} \text{cov}(a)}{\sum\limits_{\substack{\text{arcs } a \\ \text{mult}(a)>0}} \text{mult}(a)}$$

$$(1)$$

$$\hat{\lambda}^{\text{node}}_{\text{err}} = \frac{\sum\limits_{\substack{\text{nodes } n \\ \text{mult}(n)=0}} \text{cov}(n)}{\sum\limits_{\substack{\text{nodes } n \\ \text{mult}(n)=0}} |\text{k-mers}(n)|} \qquad \hat{\lambda}^{\text{arc}}_{\text{err}} = \frac{\sum\limits_{\substack{\text{arcs } a \\ \text{mult}(a)=0}} \text{cov}(a)}{\sum\limits_{\substack{\text{arcs } a \\ \text{mult}(a)=0}} 1}$$

$$(2)$$

wherein the weights $w_m$ are calculated as the number of nodes/arcs assigned with multiplicity m up until a certain tuneable multiplicity $m'$. All weights $w_m$, $m > m'$ may be calculated as a fraction of $w_{m'}$ : $w_m = \beta^{(m-m')}w^{m'}$. $\beta < 1$ is determined as $w_{m'}/w_{m'-1}$ because most nodes will have a low multiplicity and modelling higher multiplicity weights by e.g. a geometric distribution provides good results. To determine the overdispersion factor $f$, the sample variance for each multiplicity $m \leq m'$ is calculated separately as

$$\hat{S}_m^{\text{node}} = \frac{1}{|\text{mult}(n) = m|} \sum_{\substack{\text{nodes } n \\ \text{mult}(n)=m}} \left( \frac{\text{cov}(n)}{|\text{k-mers}(n)|} - m\lambda^{\text{node}} \right)^2$$

$$\hat{S}_m^{\text{arc}} = \frac{1}{|\text{mult}(a) = m|} \sum_{\substack{\text{arcs } a \\ \text{mult}(a)=m}} \left( \text{cov}(a) - m\lambda^{\text{arc}} \right)^2 .$$

The factor f may be calculated by taking a weighted mean of the sample variances based on the number of nodes/arcs that are assigned with a certain multiplicity:

$$f^{\text{node}} = \frac{1}{\sum_{m=0}^{m'} w_m} \sum_{m=0}^{m'} w_m \frac{\hat{S}_m^{\text{node}}}{m\lambda^{\text{node}}}$$

$$f^{\text{arc}} = \frac{1}{\sum_{m=0}^{m'} w_m} \sum_{m=0}^{m'} w_m \frac{\hat{S}_m^{\text{arc}}}{m\lambda^{\text{arc}}}$$

Table 1 shows results obtained by the above described method. The method was performed on reads obtained from a S.enterica genome for different read coverages (Coverage) and for different sizes of the CRF (CRF-size). The simulation where the CRF-size is zero corresponds to the case where only the k-mer spectrum static thresholds are used. All results were compared with a reference genome of the S.enterica to obtain an accuracy (Acc) measure for each simulation.

**Table 1: Results**

| Coverage | 10 | | 15 | | 20 | | 30 | | 50 | | 75 | | 90 | |
|----------|------|------|------|------|------|------|------|------|------|------|------|------|------|------|
| CRF-size | Acc | Iter | Acc | Iter | Acc | Iter | Acc | Iter | Acc | Iter | Acc | Iter | Acc | Iter |
| 0 | 80.24 | 25 | 87.14 | 25 | 86.79 | 25 | 87.33 | 7 | 94.76 | 10 | 96.43 | 10 | 96.99 | 11 |
| 4 | 86.57 | 10 | 92.78 | 11 | 93.99 | 10 | 95.20 | 10 | 96.31 | 8 | 97.10 | 8 | 97.41 | 7 |
| 8 | 87.02 | 9 | 93.02 | 9 | 94.26 | 7 | 95.40 | 7 | 96.44 | 7 | 97.19 | 7 | 97.47 | 6 |

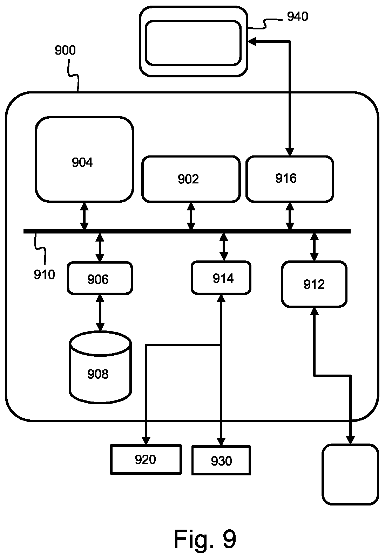

Fig. 9 shows a suitable computing system 900 enabling to implement embodiments of the above described method according to the invention. Computing system 900 may in general be formed as a suitable general-purpose computer and comprise a bus 910, a processor 902, a local memory 904, one or more optional input interfaces 914, one or more optional output interfaces 916, a communication interface 912, a storage element interface 906, and one or more storage elements 908. Bus 910 may comprise one or more conductors that permit communication among the components of the computing system 900. Processor 902 may include any type of conventional processor or microprocessor that interprets and executes programming instructions. Local memory 904 may include a random-access memory (RAM) or another type of dynamic storage device that stores information and instructions for execution by processor 902 and/or a read only memory (ROM) or another type of static storage device that stores static information and instructions for use by processor 902. Input interface 914 may comprise one or more conventional mechanisms that permit an operator or user to input information to the computing device 900, such as a keyboard 920, a mouse 930, a pen, voice recognition and/or biometric mechanisms, a camera, etc. Output interface 916 may comprise one or more conventional mechanisms that output information to the operator or user, such as a display 940, etc. Communication interface 912 may comprise any transceiver-like mechanism such as for example one or more Ethernet interfaces that enables computing system 900 to communicate with other devices and/or systems. The communication interface 912 of computing system 900 may be connected to such another computing system by means of a local area network (LAN) or a wide area network (WAN) such as for example the internet. Storage element interface 906 may comprise a storage interface such as for example a Serial Advanced Technology Attachment (SATA) interface or a Small Computer System Interface (SCSI) for connecting bus 910 to one or more storage elements 908, such as one or more local disks, for example SATA disk drives, and control the reading and writing of data to and/or from these storage elements 908. Although the storage element(s) 908 above is/are described as a local disk, in general any other suitable computer-readable media such as a removable magnetic disk, optical storage media such as a CD or DVD, -ROM disk, solid state drives, flash memory cards, ... could be used.

As used in this application, the term "circuitry" may refer to one or more or all of the following:

(a) hardware-only circuit implementations such as implementations in only analog and/or digital circuitry and
(b) combinations of hardware circuits and software, such as (as applicable):

(i) a combination of analog and/or digital hardware circuit(s) with software/firmware and
(ii) any portions of hardware processor(s) with software (including digital signal processor(s)), software, and memory(ies) that work together to cause an apparatus, such as a mobile phone or server, to perform various functions) and

(c) hardware circuit(s) and/or processor(s), such as microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in a server, a cellular network device, or other computing or network device.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the scope of the claims are therefore intended to be embraced therein.

It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

**Claims**

1.  A computer-implemented method for processing reads (110, 310, 410, 502) of a sequenced nucleic acid (100, 210, 500) comprising the following steps:

- determining (503) k-mers (321, 421, 504, 551) from the reads;
- determining (505) first counts (553) indicative for the respective number of occurrences of the respective k-mers (551) in the reads, and second counts (554) indicative for the respective number of overlaps (560) between respective k-mers in the reads;
- determining (507) a k-mer spectrum (530) from the first counts;
- determining (507) first output counts (653) indicative for the respective number of occurrences (651) of the respective k-mers in the nucleic acid based on a position (531, 532, 533) of the k-mers in the k-mer spectrum (530) and based on a constraint (509, 510) comprising:

   ◦ a first output count of a respective k-mer equals the total number of overlaps of the respective k-mer with other k-mers in the nucleic acid; and/or
   ◦ a first output count of a respective k-mer equals the total number of overlaps of other k-mers with the respective k-mer.

2. The method according to claim 1 further comprising determining (507) second output counts (654) indicative for the respective number of overlaps (660) between respective k-mers in the nucleic acid based on the position (531, 532, 533) of the k-mers in the k-mer spectrum (530) and based on the constraint (509, 510).

3. The method according to claim 1 or 2 wherein the determining (507) the first and, when dependent on claim 2, second output counts (653, 654) further comprises assigning probabilities for candidate output counts to the k-mers.

4. The method according to claim 3 wherein a candidate output count is assigned a lower probability when violating the constraint (509, 510).

5. The method according to any one of the preceding claims wherein the determining (505) the first and second counts further comprises determining a graph (550) comprising nodes (551, 555, 558) and directed edges (557, 556, 552, 560, 559); and wherein the k-mers (504) are represented by the respective nodes of the graph and the overlaps are represented by the respective edges of the graph.

6. The method according to any one of the preceding claims wherein the determining (505) the first and, when depending on claim 2, second output counts is performed by a probabilistic graphical model (870).

7. The method according to claim 6 wherein the probabilistic graphical model is a conditional random field, CRF, model (870).

8. The method according to claim 6 or 7 wherein the model (870) comprises the first output counts (653) and, when dependent on claim 2, second output counts (654) as unobserved variables.

9. The method according to any one of claims 6, 7 and 8 wherein the model (870) comprises the first and/or second counts as observed variables.

10. The method according to any one of claims 6 to 9 wherein the determining the first output counts and, when depending on claim 2, the second output counts further comprises selecting a neighbourhood (860) of a respective k-mer and constructing a probabilistic graphical model (870) of the neighbourhood and determining the first output count and/or, when depending on claim 2, the second output count associated with the respective k-mer therefrom.

11. The method according to any one of the preceding claims wherein the overlaps between the k-mers are k-1 overlaps.

12. A data processing system configured to perform the method according to any one of claims 1 to 11.

13. A computer program comprising instructions which, when the program is executed by a computer, cause the computer to perform the method according to any one of claims 1 to 11.

14. A computer-readable medium comprising instructions which, when executed by a computer, cause the computer to perform the method according to any one of claims 1 to 11.

Fig. 1

ACATAGCATGCAG

210

Fig. 2

ATAGCA
ACATAG
ATGCAG
AGCATG
...
CATGCA

310

Fig. 3

ATAGCT
ACATAG
ATACAG
AGCATG
...
CATGCT

410

Fig. 4

EP 3 675 129 A1

12

EP 3 675 129 A1

Fig. 5

500

OBTAIN READS
501

ATAGCT
ACATAG
...
CATGCT
502

DETERMINE K-MERS
503

ATA TAG
AGC GCT
... TCC
504

DETERMINE ARC AND
NODE COUNTS
505

DETERMINE ARC AND
NODE MULTIPLICITIES
507

SPECTRUM
508

NODE MULTIPLICITY =
SUM(INCOMING ARC
MULTIPLICITIES)
509

NODE MULTIPLICITY =
SUM(OUTGOING ARC
MULTIPLICITIES)
510

550
557
4
555
TAC
12
3
556
4
573
552
553
ACA
11
20
11
ATA
14
10
TAG
551
CAT
10
572
8
8
8
9
16
554
ATG
GCA
8
AGC
10
560
7
8
561
TGC
8
2
559
2
1
571
GCT
2
558
CAG
3
580
570

530

0.4
531
534
532
533
0.3
0.2
535
536 537 538
0.1
0
0 5 10 15 20 25 30

13

Fig. 6

Fig. 7

EP 3 675 129 A1

Fig. 8

Fig. 9

Europäisches
Patentamt

European
Patent Office

Office européen
des brevets

**EUROPEAN SEARCH REPORT**

Application Number

EP 18 24 8105

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PHILLIP E C COMPEAU ET AL: "Why are de Bruijn graphs useful for genome assembly?", NATURE BIOTECHNOLOGY, vol. 29, no. 11, 27 July 2017 (2017-07-27) , pages 987-991, XP055598024, New York ISSN: 1087-0156, DOI: 10.1038/nbt.2023 | 1-5 | INV. G16B30/00 |
| A | * abstract * * page 3, paragraph 3 - paragraph 5 * * page 9, paragraph 1 - paragraph 2 * | 6-14 | |
| X | PEVZNER PAVEL A ET AL: "Fragment assembly with double-barreled data", BIOINFORMAT, OXFORD UNIVERSITY PRESS, SURREY, GB, vol. 17, no. suppl. 1, 1 June 2001 (2001-06-01), pages s225-s233, XP008140748, ISSN: 1367-4803, DOI: 10.1093/BIOINFORMATICS/17.SUPPL_1.S225 | 1-5 | |
| A | * abstract * * page S228, column 1, paragraph 3 * * page S232, column 1, paragraph 4 - column 2, paragraph 2 * | 6-14 | TECHNICAL FIELDS SEARCHED (IPC) G16B |
| A | ANTOINE LIMASSET ET AL: "Toward perfect reads: self-correction of short reads via mapping on de Bruijn graphs", ARXIV.ORG, CORNELL UNIVERSITY LIBRARY, 201 OLIN LIBRARY CORNELL UNIVERSITY ITHACA, NY 14853, 9 November 2017 (2017-11-09), XP081319636, * abstract * * page 2, column 1, paragraph 3 * | 1-14 | |

-/--

The present search report has been drawn up for all claims

| Place of search | Date of completion of the search | Examiner |
|---|---|---|
| Munich | 24 June 2019 | Schmitt, Constanze |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 18 24 8105

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SARA EL-METWALLY ET AL: "Next-Generation Sequence Assembly: Four Stages of Data Processing and Computational Challenges", PLOS COMPUTATIONAL BIOLOGY, vol. 9, no. 12, 12 December 2013 (2013-12-12), page e1003345, XP055597988, DOI: 10.1371/journal.pcbi.1003345 * abstract * * page 5, column 1, paragraph 2 * * page 7, column 1, paragraph 1 * ----- | 1-14 | |

TECHNICAL FIELDS
SEARCHED (IPC)

The present search report has been drawn up for all claims

| Place of search | Date of completion of the search | Examiner |
|---|---|---|
| Munich | 24 June 2019 | Schmitt, Constanze |

EPO FORM 1503 03.82 (P04C01)